# EUROPÄISCHE PATENTANMELDUNG

(11) **EP 4 306 492 A1**
(43) Veröffentlichungstag der Anmeldung: **17.01.2024**
(21) Anmeldenummer: 22184922.7
(22) Anmeldetag: 14.07.2022
(51) Int. Cl.: C03C 4/00, C03C 10/00

(54) **GLASKERAMIK-FORMKÖRPER FÜR DENTALE ZWECKE**

(71) Anmelder: VITA-ZAHNFABRIK H. Rauter GmbH & Co. KG, 79713 Bad Säckingen (DE)
(72) Erfinder: Neun, Christopher, 79713 Bad Säckingen (DE); Gödiker, Michael, 79713 Bad Säckingen (DE)
(74) Vertreter: dompatent von Kreisler Selting Werner - Partnerschaft von Patent- und Rechtsanwälten mbB

(57) **Zusammenfassung**

Die vorliegende Erfindung betrifft einen Glaskeramik-Formkörper für dentale Zwecke mit einem sich graduell und kontinuierlich veränderndem Gewichtsverhältnis von amorphem Anteil zu kristallinem Anteil, ein Verfahren zu seiner Herstellung sowie seine Verwendung zur Herstellung dentaler Restaurationen.

## Beschreibung

Die vorliegende Erfindung betrifft einen Glaskeramik-Formkörper für dentale Zwecke mit einem sich graduell und kontinuierlich verändernden Gewichtsverhältnis von amorphem Anteil zu kristallinem Anteil, ein Verfahren zu seiner Herstellung sowie seine Verwendung zur Herstellung dentaler Restaurationen.

Die Anforderungen, die an modernes Zahnersatzmaterial gestellt werden, sind vielfältig. So sollte die Fertigung der individuellen Restauration, von denen jede ein Unikat ist, mit höchster Präzision und einem vertretbaren Aufwand möglich sein. Zudem sollte sich die Restauration optisch ähnlich transluzent wie Zahnschmelz verhalten und sich farblich in die Reihe der natürlichen Zähne adaptieren. In anderen Fällen muss das Restaurationsmaterial dagegen opak sein, um damit beispielsweise einen verfärbten Zahnstumpf abdecken zu können. Schließlich muss das Material langfristig den Kaukräften standhalten, während es gleichzeitig einem ständigen Angriff von Säuren ausgesetzt ist.

Glaskeramiken haben sich als gängiges Material für die Herstellung dentaler Restaurationen etabliert, wobei vor allem ihre Festigkeit und die ästhetischen Eigenschaften geschätzt werden. Durch den kristallinen Anteil wird nicht nur die Ausbreitung von Rissen unterbunden, sondern auch das Licht anders reflektiert und abgelenkt als bei klassischen Gläsern. Dadurch entsteht eine dem natürlichen Zahn sehr nahekommende Transluzenz, weshalb dentale Glaskeramiken besonders in der ästhetischen Zone im Frontzahnbereich eingesetzt werden.

Auch wenn Glaskeramiken als Zahnersatzmaterial bereits gute optische Eigenschaften mitbringen, bleibt die farbliche Anpassung an das natürliche Zahnbild weiterhin eine Herausforderung.

EP 2 765 119 beschreibt einen Rohling für dentale Zwecke, der mindestens 2 miteinander verbundene Schichten von Lithiumsilikat-Glas, Lithiumsilikat-Glas mit Keimen oder Lithiummetasilikat-Glaskeramik aufweist, wobei die Schichten sich in der Farbe unterscheiden und die Schichten monolithisch sind. So soll es möglich sein, optische Eigenschaften von natürlichem Zahnmaterial gut nachzuahmen und eine Formgebung ohne Schrumpf zu erreichen.

WO 2013/086187 betrifft eine Lithiumsilikat-Glaskeramik, die 6 bis 30 Gew.-% Cs₂O, 55 bis 80 Gew.-% SiO₂, 1 bis 5 Gew.-% Al₂O₃ und B₂O₃, 7 bis 16 Gew.-% Li₂O und 1 bis 5 Gew.-% P₂O₅ aufweist, wobei sich die Angaben in Gew.-% jeweils auf das Gesamtgewicht der Glaskeramik beziehen. Mit dieser Zusammensetzung sollen sich insbesondere Blöcke mit einer hohen Transparenz erhalten lassen.

Obwohl im Stand der Technik einige Anstrengungen unternommen wurden, Glaskeramiken zur Verfügung zu stellen, die den ästhetischen Anforderungen von dentalen Restaurationen nahekommen, besteht weiterhin der Bedarf nach verbesserten Materialien, insbesondere solchen, die den natürlichen Farbverlauf eines Zahns ohne erkennbare Übergänge zwischen Inzisal-, Dentin- und Zervikalbereich nachbilden können.

Vor diesem Hintergrund liegt der vorliegenden Erfindung die Aufgabe zugrunde, ein Material für die Herstellung dentaler Restaurationen zur Verfügung zu stellen, mit dem die optische Erscheinung von natürlichem Zahnmaterial nachgeahmt werden kann.

Diese Aufgabe wird durch einen Formkörper gelöst, der sich an dem Konzept der functional graded materials (FGM) orientiert, mit dem sich überraschender Weise ein Material für dentale Restaurationen mit einer hohen Ästhetik bei gleichzeitig vergleichsweisen geringen Produktionskosten erzielen lässt.

Daher ist ein erster Gegenstand der vorliegenden Erfindung ein Glaskeramik-Formkörper für dentale Zwecke umfassend einen amorphen Anteil und einen kristallinen Anteil, dadurch gekennzeichnet, dass der Formkörper ein sich kontinuierlich und graduell veränderndes Gewichtsverhältnis von amorphem zu kristallinem Anteil aufweist.

Sofern nicht anders angegeben, kann das Gewichtsverhältnis des amorphen und kristallinen Anteils sowie die Zusammensetzung der Kristallphasen mittels Rietveld-Analyse bestimmt werden mit MgO als internem Standard.

In einer besonders bevorzugten Ausführungsform bildet das Gewichtsverhältnis von amorphem Anteil zu kristallinem Anteil im Formkörper einen Gradienten aus. Vorzugsweise bewegt sich das Gewichtsverhältnis von amorphem zu kristallinem Anteil dabei im Bereich von 65:35 bis 35:65, vorzugsweise 60:40 zu 40:60.

Gradient im Sinne der vorliegenden Erfindung bezeichnet, den Verlauf einer graduellen und kontinuierlichen Änderung einer Größe auf einer bestimmten Strecke. Bei der Größe kann es sich beispielsweise um das Gewichtsverhältnis von amorphem Anteil zu kristallinem Anteil, aber auch um physikalische Eigenschaften handeln.

Herkömmliche Formkörper, die einen natürlichen Farbverlauf des Zahns nachbilden wollen, weisen meist den Nachteil auf, dass dieser durch einen schichtweisen Aufbau des Formkörpers erreicht wird, wobei sich Farbübergänge zwischen den Schichten negativ auf das optische Erscheinungsbild auswirken können. Im Gegensatz dazu wurde im Rahmen der vorliegenden Erfindung überraschend gefunden, dass ein solcher Farbverlauf auch für einen monolithischen Formkörper realisieren lässt, wodurch ein ansatzloser Übergang im Farbverlauf erzielt wird. Daher ist eine Ausführungsform der vorliegenden Erfindung bevorzugt, in der Formkörper monolithisch ist.

Monolithisch im Sinne der vorliegenden Erfindung bezeichnet einen einstückigen, aus einem Stück bestehenden Formkörper, der frei von Schichten und/oder Grenzflächen ist.

Es wurde überraschend gefunden, dass mit solchen Formkörpern eine dentale Restauration mit einem deutlich natürlicheren Erscheinungsbild erhalten werden kann, die ohne aufwendige Bearbeitung in das natürliche Zahnschema eingefügt werden kann.

Ohne an eine bestimmte Theorie gebunden zu sein, wird davon ausgegangen, dass Gradient, der durch das Gewichtsverhältnis von amorphem Anteil zu kristallinem Anteil gebildet wird, dass auch andere Eigenschaften des Formkörpers einem Gradienten folgen. In einer bevorzugten Ausführungsform weist der erfindungsgemäße Formkörper daher einen Gradienten bezüglich mindestens einer der Eigenschaften auf, die ausgewählt sind aus der Gruppe bestehend aus Transmissionsgrad, Vickers-Härte, insbesondere HV3, Festigkeit, L^{∗}a^{∗}b-Werte und Risszähigkeit.

Der erfindungsgemäße Formkörper zeichnet sich insbesondere dadurch aus, dass er wie von "functionally graded materials" bekannt, Gradienten bezüglich verschiedener seiner Eigenschaften aufweist. In einer bevorzugten Ausführungsform ist der erfindungsgemäße Formkörper dadurch gekennzeichnet, dass er keine Grenzfläche an einem Punkt aufweist, an dem sich ein Gradientwert einer Eigenschaft ändert.

Der erfindungsgemäße Formkörper zeichnet sich insbesondere durch seine optischen Eigenschaften aus, die für eine Verwendung für dentale Zwecke abgestimmt sind. In diesem Zusammenhang ist eine Ausführungsform bevorzugt, in der der Formkörper einen Transmissionsgrad für Licht mit einer Wellenlänge von 360 bis 740 nm von 20 bis 60%, vorzugsweise 25 bis 50% aufweist, bestimmt an 1 mm dicken Probenkörpern.

Der erfindungsgemäße Formkörper ist insbesondere für die Herstellung dentaler Restaurationen vorgesehen und muss entsprechend über eine ausreichende Festigkeit zur Bearbeitung verfügen, aber auch stabil genug sein, um den Kaukräften standzuhalten. Daher ist eine Ausführungsform bevorzugt, in der der erfindungsgemäße Formkörper eine Festigkeit von 200 bis 600 MPa, insbesondere 300 bis 450 MPa aufweist, bestimmt gemäß DIN EN ISO 6872 im 3-Punkt-Biegeversuch.

In einer weiterhin bevorzugten Ausführungsform weist der erfindungsgemäße Formkörper eine Härte nach Vickers, insbesondere HV3, von 550 bis 800, vorzugsweise 600 bis 750 auf, bestimmt gemäß ISO EN 6507. In einer besonders bevorzugten Ausführungsform zeichnet sich der erfindungsgemäße Formkörper einen Gradienten bezüglich seiner Härte aufweist. Die Differenz der Vickershärte im erfindungsgemäßen Formkörper entlang des Gradienten beträgt vorzugsweise 10 bis 100, besonders bevorzugt 20 bis 80.

Um eine gute Passgenauigkeit der dentalen Restauration zu erreichen, kann diese mittels CAD/CAM-Verfahren hergestellt werden, was voraussetzt, dass der Formkörper subtraktiv bearbeitet werden kann, beispielsweise mittels Fräsen. Um Rissbildungen und Rissausbreitungen bei der Bearbeitung zu vermeiden, weist der erfindungsgemäße Rohling vorzugsweise eine Risszähigkeit von 1,0 bis 3,0 MPa^{∗}m^{1/2}, vorzugsweise 1,2 bis 2,5 MPa^{∗}m^{1/2} auf, jeweils bestimmt mittels der SEVNB-Methode.

Der Gradient des erfindungsgemäßen Formkörpers schlägt sich insbesondere in der Farbe und Transluzenz nieder, wobei diese Begriffe austauschbar sind. Die Farbe kann insbesondere durch ihren L^{∗}a^{∗}b^{∗}-Wert oder durch einen in der Dentalindustrie üblichen Farbschlüssel charakterisiert werden.

Ein natürlicher Zahn weist einen Farbverlauf auf, der ausgehend vom Zahnfleisch zur Schneide oder Kaufläche einem Gradienten folgt. Um dieses natürliche Bild nachzubilden, hat es sich als vorteilhaft erwiesen, wenn der Gradient einer Achse folgt, die durch den Formkörper verläuft. In einer bevorzugten Ausführungsform verläuft der Gradient senkrecht zur längsten Ausdehnung des Formkörpers, vorzugsweise 90° zu dessen Längsachse. In einer alternativ bevorzugten Ausführungsform verläuft der Gradient entlang der längsten Ausdehnung des Formkörpers, vorzugsweise parallel zu dessen Längsachse. Hier hat sich gezeigt, dass dieser Verlauf besonders vorteilhaft für die maschinelle Bearbeitung des Formkörpers mit einer Fräs- und/oder Schleifmaschine ist.

Der Gradient im Formkörper wird durch das sich kontinuierlich ändernde Gewichtsverhältnis von amorphem zu kristallinem Anteil erreicht. Darüber hinaus ist eine Ausführungsform bevorzugt, in der der kristalline Anteil unterschiedliche Phasen aufweist, wobei sich wenigstens zwei der Phasen voneinander unterscheiden. Auf diese Weise können die optischen Eigenschaften des erfindungsgemäßen Formkörpers weiter angepasst und optimiert werden. Vorzugsweise unterscheiden sich diese Phasen bezüglich wenigstens einer der folgenden Eigenschaften:
- Kristallkonzentration;
- Kristallgröße;
- Kristallform;
- Kristallzusammensetzung;
- Kristallart;
- Kristallstruktur.

Die aufgeführten Parameter beeinflussen die optischen Eigenschaften und können daher als Stellschrauben für weitere Anpassungen verwendet werden. Durch Variation der Parameter kann somit das Erscheinungsbild des erfindungsgemäßen Formkörpers individuell eingestellt werden.

In einer besonders bevorzugten Ausführungsform bilden die Phasen kristallinen Anteils einen Gradienten im Formkörpers aus.

Der kristalline Anteil des erfindungsgemäßen Formkörpers weist vorzugsweise Kristalle auf, die ausgewählt sind aus der Gruppe bestehend aus Lithiummetasilikat, Lithiumdisilikat, Lithiumphosphat, Lithium-Aluminium-Oxid, Spodumen, Virgilit, Keatit, SiO₂-Polymorphe, α-Quarz, β-Quarz, α-Tridymit, β-Tridymit, α-Cristobalit, β-Cristobalit und Mischungen hiervon, wobei Lithiummetasilikat und Lithiumdisilikat besonders bevorzugt sind. In einer besonders bevorzugten Ausführungsform weist der erfindungsgemäße Formkörper Lithiummetasilikat als dominante Kristallphase auf. In einer weiterhin bevorzugten Ausführungsform ist der Formkörper vorzugsweise frei von Spodumen und/oder Virgilit, wobei deren Anteil im Formkörper vorzugsweise weniger als 1 Gew.-%, besonders bevorzugt weniger als 0,5 Gew.-%, insbesondere weniger als 0,1 Gew.-% und im Speziellen weniger als 0,01 Gew.-% beträgt, jeweils bezogen auf das Gesamtgewicht des Formkörpers.

Der erfindungsgemäße Formkörper zeichnet sich insbesondere durch seine optischen Eigenschaften aus. Es wurde überraschend gefunden, dass sich diese besonders vorteilhaft ausbilden, wenn sich der amorphe Anteil der im Formkörper in gewissen Bereichen bewegt. Daher ist eine Ausführungsform bevorzugt, in der der amorphe Anteil im Formkörper 30 bis 70 Gew.-%, vorzugsweise 40 bis 60 Gew.-%beträgt, jeweils bezogen auf das Gesamtgewicht des Formkörpers. Auf diese Weise kann insbesondere ein Transluzenzverlauf erreicht werden, der dem eines natürlichen Zahns entspricht.

Um das Erscheinungsbild eines natürlichen Zahns nachzuempfinden, hat es sich als vorteilhaft erwiesen, wenn sich der amorphe Anteil nur in einem geringen Umfang im Formkörper ändert, wobei jeweils ein amorpher Mindestanteil bevorzugt ist. In einer bevorzugten Ausführungsform ändert sich daher der amorphe Anteil im Formkörper entlang des Gradienten um wenigstens 5 Gew.-%, vorzugsweise um wenigstens 7 Gew.-%, aber vorzugsweise um nicht mehr als 30 Gew.-%, jeweils bezogen auf das Gesamtvolumen des Formkörpers. In einer besonders bevorzugten Ausführungsform liegt die Änderung des amorphen Anteils im Formkörper entlang des Gradienten im Bereich von 5 bis 30 Gew.-%, vorzugsweise 15 bis 25 Gew.-%, jeweils bezogen auf das Gesamtgewicht des Formkörpers.

Die Eigenschaften des Formkörpers können unter anderem durch den Gehalt an Lithiumdisilikat und Lithiummetasilikat im kristallinen Anteil des Formkörpers erreicht werden kann. In einer bevorzugten Ausführungsform ändert sich der Gehalt an Lithiumdisilikat (Li₂Si₂O₅) im Formkörper daher entlang des Gradienten um mindestens 5 Gew.-%, vorzugsweise um mindestens 7 Gew.-%, besonders bevorzugt um 5 bis 20 Gew.-%, insbesondere um 7 bis 15 Gew.-%, bezogen auf das Gesamtgewicht des kristallinen Anteils.

In einer weiterhin bevorzugten Ausführungsform ändert sich der Gehalt an Lithiummetasilikat (Li₂SiO₃) im Formkörper entlang des Gradienten um maximal 15 Gew.-%, vorzugsweise um maximal 10 Gew.-%, besonders bevorzugt um 1 bis 15 Gew.-%, insbesondere um 3 bis 10 Gew.-%, ändert, bezogen auf das Gesamtgewicht des kristallinen Anteils.

In einer besonders bevorzugten Ausführungsform weist der kristalline Anteil einen Gradienten bezüglich seiner Zusammensetzung auf. Dabei kann sich die Zusammensetzung beispielsweise bezüglich des Verhältnisses von Lithiummetasilikat zu Lithiumdisilikat ändern. In einer besonders bevorzugten Ausführungsform weist der Formkörper einen Gradienten der Zusammensetzung, der von einem Gewichtsverhältnis von Li₂SiO₃/Li₂Si₂O₅ von 50:50 bis 100:0 reicht.

Bei dem erfindungsgemäßen Formkörper handelt es sich vorzugsweise um eine Lithiumsilikat-Keramik. Daher ist eine Ausführungsform bevorzugt, in der die Zusammensetzung des Formkörpers 50 bis 75 Gew.-%, vorzugsweise 55 bis 70 Gew.-%, insbesondere 58 bis 66 Gew.-% SiO₂ aufweist, jeweils bezogen auf das Gesamtgewicht.

Weiterhin ist eine Ausführungsform bevorzugt, in der die Zusammensetzung des Formkörpers 10 bis 20 Gew.-%, Li₂O aufweist, bezogen auf das Gesamtgewicht des Formkörpers.

Vorzugsweise beträgt das molare Verhältnis von Li₂O:SiO₂ im Formkörper 1,5 bis 2,5, was sich als besonders vorteilhaft zur Ausbildung einer Lithiumsilikat-Glaskeramik erwiesen hat.

Es wurde überraschend gefunden, dass der im erfindungsgemäßen Formkörper vorgesehene Verlauf des Verhältnisses von Glasphase zu kristalliner Phase sowie die unterschiedlichen Domänen der Kristallphase durch spezielle Wärmebehandlungen erreicht werden können. Daher ist ein weiterer Gegenstand der vorliegenden Erfindung ein Verfahren zur Herstellung eines erfindungsgemäßen Formkörpers, das die folgenden Schritte umfasst:
a) Bereitstellen eines glaskeramischen Rohlings, der durch Wärmebehandlung zur Ausbildung von Kristallen befähigt ist, und
b) Inhomogene Wärmebehandlung des Rohlings unter Erhalt des Formkörpers.

Die inhomogene Wärmebehandlung des Rohlings umfasst vorzugsweise wenigstens eine oder mehrere inhomogene Wärmebehandlung(en), die durch Einstellen eines isothermen und / oder nicht-isothermen Wärmegradienten erfolgt. In einer besonders bevorzugten Ausführungsform umfasst das erfindungsgemäße Verfahren weiterhin eine homogene Wärmebehandlung, die sich vorzugsweise an die inhomogene Wärmebehandlung in Schritt b) anschließt.

Um die inhomogene Wärmebehandlung des Rohlings zu erreichen, wird vorzugsweise ein erster Bereich des Rohlings bei einer Temperatur T₁ und ein zweiter Bereich des Rohlings bei einer Temperatur T₂ behandelt, wobei die Temperaturdifferenz zwischen Temperatur T₁ und Temperatur T₂ mindestens 5 °C, bevorzugt mindestens 10 °C, weiter bevorzugt mindestens 40 °C oder mindestens 50 °C oder mindestens 75 °C oder mehr beträgt und/oder die Temperatur T₂ vorzugsweise höher ist als die Temperatur T₁.

Die Temperatur T₁ liegt hierbei bevorzugt zwischen 600 °C und 750 °C, besonders bevorzugt im Bereich zwischen 650 °C und 720 °C, während die Temperatur T₂ bevorzugt zwischen 700 °C und 900 °C, besonders bevorzugt zwischen 750 °C und 850 °C, liegt.

Die inhomogene Wärmebehandlung des Rohlings erfolgt vorzugsweise durch Kontakt mit einer Wärmequelle, wobei der Kontakt direkt oder indirekt sein kann. Bei der Wärmequelle kann es sich um einen Wärmeraum oder eine beheizbare Unterlage handeln.

In einer bevorzugten Ausführungsform erfolgt die Wärmehandlung dadurch, dass der Rohling zumindest teilweise, vorzugsweise formschlüssig, in einen Wärmeraum eingebracht und der Wärmeraum erwärmt wird. Auf diese Weise kann eine gezielte Wärmebehandlung einzelner Bereiche des Rohlings erreicht werden. Bei dem Wärmeraum handelt es sich vorzugsweise um ein wärmeleitfähiges Material mit einer Wärmeleitfähigkeit von 50 bis 500 W/(m^{∗}K), vorzugsweise 150 bis 450 W/(m^{∗}K), bestimmt gemäß Wärmestromkalorimetrie.

Das wärmeleitende Material ist vorzugsweise ausgewählt aus der Gruppe bestehend aus Nichtoxidkeramiken, vorzugsweise ausgewählt aus Si₃N₄, Siliziumkarbid und Aluminiumnitrid, oder Metallen.

Die Erwärmung des Wärmeraums kann je nach Intensität angepasst werden. In einer bevorzugten Ausführungsform wird der Wärmeraum daher indirekt durch die Umgebungsatmosphäre oder besonders bevorzugt direkt über Kontakt mit einer Wärmequelle erwärmt.

Alternativ und besonders bevorzugt kann die Wärmebehandlung des Rohlings auch durch eine beheizbare Unterlage erfolgen, auf die der Rohling gestellt wird. Auch hier kann je nach gewünschter Intensität der Kontakt indirekt oder direkt sein. Daher ist eine Ausführungsform bevorzugt, in der es sich bei der beheizbaren Unterlage um eine Heizplatte handelt, auf die der Rohling gestellt wird. Alternativ kann es sich bei der beheizbaren Unterlage um ein wärmeleitendes Material handeln, das beispielsweise über eine Heizplatte erwärmt wird, so dass die Erwärmung des Rohlings indirekt erfolgt.

Es können auch beide Arten der Wärmebehandlung miteinander kombiniert werden.

Ein weiterer Gegenstand der vorliegenden Erfindung ist die Verwendung des erfindungsgemäßen Formkörpers zur Herstellung dentaler Restaurationen. Vorzugsweise wird der erfindungsgemäße Formkörper zur Herstellung von dentalen Restaurationen im Frontzahnbereich, insbesondere Veneers, Kronen, Inlays und Onlays verwendet.

Ein weiterer Gegenstand der vorliegenden Erfindung ist ein Verfahren zur Herstellung einer dentalen Restauration unter Verwendung des erfindungsgemäßen Formkörpers, wobei im Rahmen des erfindungsgemäßen Verfahrens ein erfindungsgemäßer, Formkörper, der mindestens einer ersten inhomogenen Wärmebehandlung unterzogen wurde, bereitgestellt wird und einer weiteren, isothermen Wärmebehandlung unterzogen wird. Die Temperatur dieser isothermen Wärmebehandlung liegt hierbei bevorzugt zwischen 730 und 850 °C, besonders bevorzugt im Bereich zwischen 750 °C und 820 °C. Die Haltedauer bei dieser Temperatur beträgt vorzugsweise zwischen einer und 15 Minuten, besonders bevorzugt zwischen 3 und 10 Minuten. Dieser Prozessschritt kann optional auch direkt im gleichen Ofenbrand an die erste inhomogene Wärmebehandlung angegliedert sein. In einer bevorzugten Ausführungsform umfasst das erfindungsgemäße Verfahren weiterhin die maschinelle Bearbeitung des Formkörpers unter Ausbildung der geometrischen Form der dentalen Restauration, vorzugsweise bevor der Formkörper der weiteren Wärmebehandlung unterzogen wird.

Die vorliegende Erfindung wird anhand der folgenden Beispiele und Figuren näher beschrieben, wobei diese jedoch keinesfalls als Einschränkung des Erfindungsgedankens zu verstehen sind.

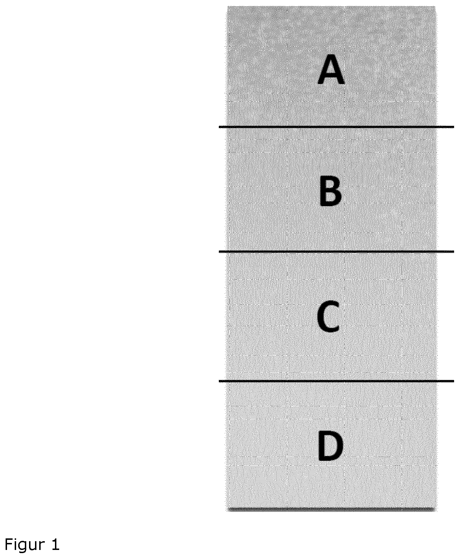

Figur 1 zeigt einen beispielhaften erfindungsgemäßen Formkörper, der einen Gradienten bezüglich des Verhältnisses des amorphen Anteils zu kristallinem Anteil als auch der Zusammensetzung des kristallinen Anteils aufweist. Tabelle 1 zeigt das Ergebnis der Rietveldanalyse des Formkörpers mit MgO als internem Standard, wobei jeweils vier gleichgroße Abschnitte A bis D an verschiedenen Stellen des Formkörpers herauspräpariert und vermessen wurden. Die Anteile sind jeweils in Gew.-% angegeben.

**Tabelle 1:**

| | amorph | Li₂SiO₃ | Li₂Si₂O₅ | Li₃PO₄ |
|---|---|---|---|---|
| A | 56 | 30 | 10 | 4 |
| B | 53 | 30 | 10 | 7 |
| C | 51 | 28 | 14 | 7 |
| D | 46 | 25 | 20 | 9 |
| Δ | -10 | -5 | 10 | +5 |

Weiterhin wurden der Transmissionsgrad und die L^{∗}a^{∗}b-Werte spektroskopisch an unterschiedlichen Stellen des Formkörpers bestimmt. Dazu wurden 1 mm dicke Abschnitte aus dem Formkörper herausgearbeitet und mit Licht einer Wellenlänge von 360 bis 740 nm vermessen. Die Ergebnisse sind in Tabelle 2 zusammengefasst:

**Tabelle 2:**

| | Transmissionsgrad [%] | L^{∗} | a^{∗} | b^{∗} | h^{∗} |
|---|---|---|---|---|---|
| 1 | 48,7 | 78,5 | 0,9 | 14,8 | 86,3 |
| 2 | 47,2 | 77,5 | 1,1 | 16,2 | 86,2 |
| 3 | 38,1 | 71,2 | 1,9 | 23,4 | 85,3 |
| 4 | 25,1 | 59,7 | 4,8 | 39,8 | 83,2 |

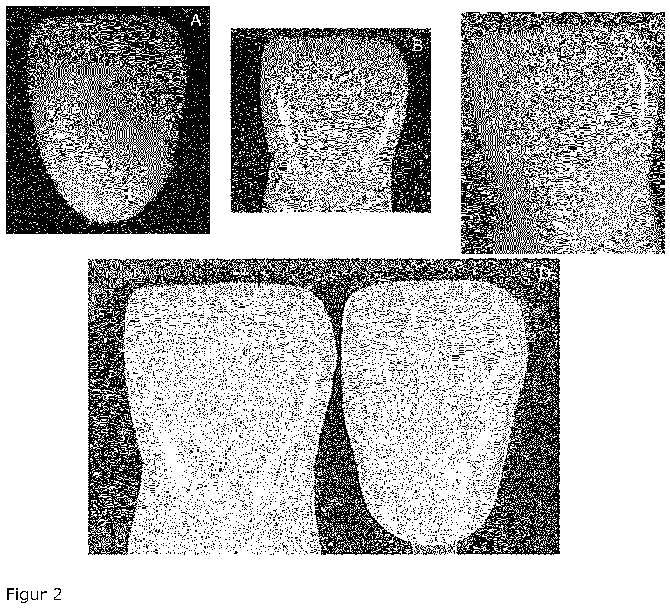

Figur 2 zeigt Abbildungen eines erfindungsgemäßen Formkörpers, wobei
A: einen erfindungsgemäßen Formkörper nach einer inhomogenen ersten Wärmebehandlung bei einer Umgebungstemperatur von 675 °C auf einer auf eine Temperatur von 790 °C aufgeheizten Unterlage zeigt;
B: einen erfindungsgemäßen Formkörper zeigt, der wie der Formkörper in Abbildung A einer inhomogenen ersten Wärmebehandlung unterzogen wurde, maschinell unter Erhalt der dentalen Restauration bearbeitet und anschließend einer homogenen Wärmehandlung bei 795 °C für 8 Minuten unterzogen wurde;
C: einen erfindungsgemäßen Formkörper zeigt, der wie der Formkörper in Abbildung A einer inhomogenen ersten Wärmebehandlung unterzogen wurde, wobei sich eine homogenen Wärmehandlung bei 795 °C für 8 Minuten anschloss. Anschließend wurde der Formkörper maschinell unter Erhalt der dentalen Restauration bearbeitet;
D: einen erfindungsgemäßer Formkörper (links) im Vergleich zu einem herkömmlichen Formkörper gemäß Vita Classical Shade Guide zeigt.

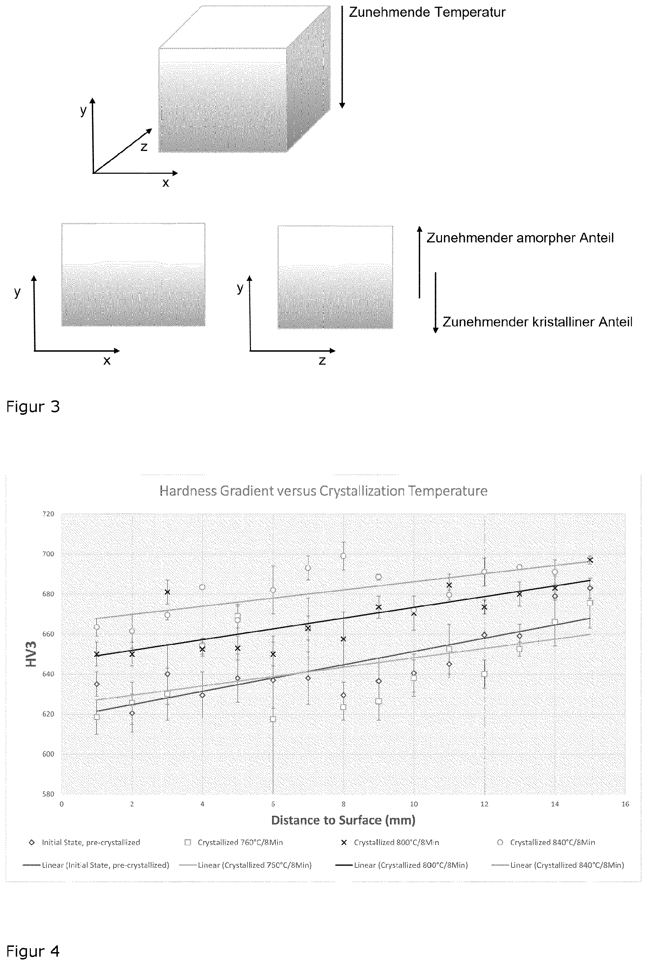

Figur 3 zeigt schematisch den graduellen Verlauf des Verhältnisses von amorphem zu kristallinem Anteil im Formkörper im Zusammenhang mit dem Temperaturverlauf während der Wärmebehandlung des Formkörpers.

Figur 4 zeigt eine Übersicht über die Vickers-Härte (HV3) eines erfindungsgemäßen Formkörpers, bestimmt an verschiedenen Stellen im Formkörper. Die Bestimmung erfolgte dabei gemäß ISO EN 6507. Wie aus der Übersicht eindeutig zu erkennen ist, weist der erfindungsgemäße Formkörper einen Gradienten bezüglich der Härte nach Vickers (HV3) auf, der sich auch bei unterschiedlichen Wärmebehandlungen einstellt.

## Patentansprüche

1. Glaskeramik-Formkörper für dentale Zwecke umfassend einen amorphen Anteil und einen kristallinen Anteil, **dadurch gekennzeichnet, dass** der Formkörper ein sich kontinuierlich und graduell veränderndes Gewichtsverhältnis von amorphem zu kristallinem Anteil aufweist.

2. Formkörper gemäß Anspruch 1, **dadurch gekennzeichnet, dass** das Gewichtsverhältnis von amorphem Anteil und kristallinem Anteil im Formkörper einen Gradienten ausbildet.

3. Formkörper gemäß wenigstens einem der vorangehenden Ansprüche, **dadurch gekennzeichnet, dass** der Formkörper monolithisch ist.

4. Formkörper gemäß wenigstens einem der vorangehenden Ansprüche, **dadurch gekennzeichnet, dass** Formkörper einen Gradienten bezüglich mindestens einer der Eigenschaften aufweist, die ausgewählt sind aus der Gruppe bestehend aus Transmissionsgrad, Festigkeit, Vickers-Härte, L^{∗}a^{∗}b-Werte und Risszähigkeit aufweist.

5. Formkörper gemäß wenigstens einem der vorangehenden Ansprüche, **dadurch gekennzeichnet, dass** der kristalline Anteil des Formkörpers unterschiedliche Kristallphasen aufweist, wobei sich wenigstens zwei der Kristallphasen kristallographisch und/oder strukturell voneinander unterscheiden.

6. Formkörper gemäß Anspruch 5, **dadurch gekennzeichnet, dass** sich die Kristallphasen bezüglich wenigstens einer der folgenden Eigenschaften unterscheiden:
∘ Kristallkonzentration;
∘ Kristallgröße;
∘ Kristallform;
∘ Kristallzusammensetzung;
∘ Kristallart;
∘ Kristallstruktur.

7. Formkörper gemäß wenigstens einem der Ansprüche 5 oder 6, **dadurch gekennzeichnet, dass** der kristalline Anteil des Formkörpers einen Gradienten bezüglich wenigstens einer der Eigenschaften aufweist.

8. Formkörper gemäß wenigstens einem der vorangehenden Ansprüche, **dadurch gekennzeichnet, dass** der amorphe Anteil im Formkörper 30 bis 70 Gew.-%, vorzugsweise 40 bis 60 Gew.-% beträgt, bezogen auf das Gesamtgewicht des Formkörpers.

9. Formkörper gemäß wenigstens einem der vorangehenden Ansprüche, **dadurch gekennzeichnet, dass** sich der amorphe Anteil im Formkörper entlang des Gradienten um mindestens 5 Gew.-%, vorzugsweise um mindestens 7 Gew.-%, besonders bevorzugt um 5 bis 30 Gew.-%, insbesondere um 15 bis 25 Gew.-%, ändert, bezogen auf das Gesamtgewicht des Formkörpers.

10. Verfahren zur Herstellung eines Formkörpers gemäß wenigstens einem der vorangehenden Ansprüche, umfassend die folgenden Schritte:
a) Bereitstellen eines glaskeramischen Rohlings, der durch Wärmebehandlung zur Ausbildung von Kristallen befähigt ist, und
b) Inhomogene Wärmebehandlung des Rohlings unter Erhalt des Formkörpers.

11. Verfahren gemäß Anspruch 10, **dadurch gekennzeichnet, dass** ein erster Bereich des Rohlings bei einer Temperatur T₁ und ein zweiter Bereich des Rohlings bei einer Temperatur T₂ behandelt wird, wobei die Temperaturdifferenz zwischen Temperatur T₁ und Temperatur T₂ mindestens 5 °C, bevorzugt mindestens 10 °C, weiter bevorzugt mindestens 40 °C oder mindestens 50 °C oder mindestens 75 °C oder mehr beträgt und/oder wobei die Temperatur T₂ vorzugsweise höher ist als die Temperatur T₁.

12. Verfahren gemäß wenigstens einem der Ansprüche 10 oder 11, **dadurch gekennzeichnet, dass** die Temperatur T₁ 600 °C und 750 °C, bevorzugt im Bereich 650 °C und 720 °C beträgt und/oder die Temperatur T₂ 700 °C und 900 °C, bevorzugt zwischen 750 °C und 850 °C beträgt.

13. Verfahren gemäß wenigstens einem der Ansprüche 10 bis 12, **dadurch gekennzeichnet, dass** die Wärmebehandlung dadurch erfolgt, dass der Rohling zumindest teilweise, vorzugsweise formschlüssig, in einen Wärmeraum eingebracht wird und der Wärmeraum erwärmt wird und/oder die Wärmebehandlung durch direkten oder indirekten Kontakt des Rohlings mit einer Wärmequelle erfolgt, wobei es sich bei der Wärmequelle vorzugsweise um eine Heizplatte handelt.

14. Verwendung eines Formkörpers gemäß wenigstens einem der Ansprüche 1 bis 9 für die Herstellung dentaler Restaurationen.

15. Verfahren zur Herstellung einer dentalen Restauration, wobei Formkörper gemäß wenigstens einem der Ansprüche 1 bis 9, der mindestens einer inhomogenen Wärmebehandlung unterzogen wurde, bereitgestellt und einer weiteren, isothermen Wärmebehandlung unterzogen wird.
